# EUROPEAN PATENT APPLICATION

(11) **EP 1 152 295 A1**
(43) Date of publication of application: **07.11.2001**
(21) Application number: 01303700.7
(22) Date of filing: 23.04.2001
(51) Int. Cl.: G03F 7/039, G03F 7/004, C08F 212/14

(54) **Tertiary-butyl acrylate polymers and their use in photoresist compositions**

(30) Priority: 05.05.2000 US 202132
(71) Applicant: Arch Specialty Chemicals, Inc., Norwalk, Connecticut 06856-4500 (US)
(72) Inventor: Malik, Sanjay, Attleboro, Massachusetts 02703 (US); Eisele, Jeff, Cranston, Rhode Island 02910 (US); Ferri, John, Cranston, Rhode Island 02910 (US); Blakeney, Andrew J., Seekonk, Massachusetts 02771 (US)
(74) Representative: Bannerman, David Gardner

(57) **Abstract**

A photosensitive resist composition comprising:
a polymer of *tertiary*-butyl acrylate having monomeric units of: 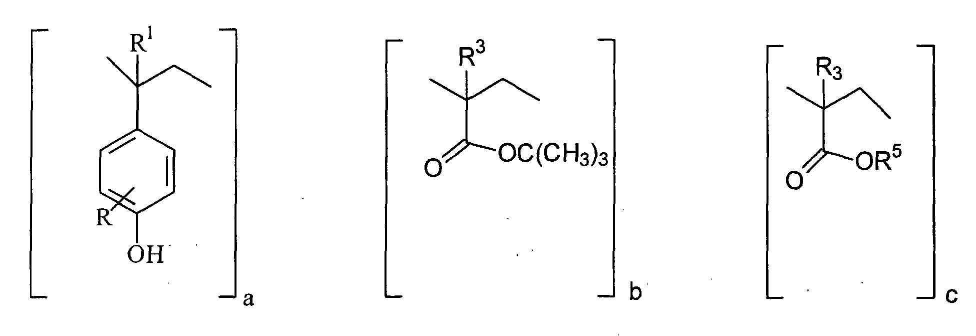
   where 0.5≤a≤0.7; 0.15≤b≤0.3; 0.1≤c≤0.2; 0.3<b+c≤0.5; R is H, or a C₁-C₄ alkyl group; R¹ = H, methyl, CH₂OR²; each R³ is independently selected from H, methyl, CH₂OR. CH₂CN, CH₂X, or CH₂COOR⁴ group
   where X= Cl, I, Br,or F; R² is H, or C₁-C₄ alkyl; R⁴ is a C₁-C₄ alkyl group; R⁵ is f a isobornyl, cyclohexyl methyl, cyclohexyl ethyl, benzyl, phenethyl, or tetrahydrofurfural group
a photoacid generator;
a solvent; and
optionally, a basic compound.

## Description

### Field of the Invention

This invention relates to a photoresist composition that has a polymer containing esters of *tertiary*-butyl acrylate as acid-labile groups to produce high resolution photoresist patterns.

### Background of the Invention

Photoresists containing copolymers of *tertiary*-butyl acrylate or methacrylate and hydroxy styrene groups are well known. *tertiary-*Butyl acrylate retards the alkaline solubility of the resist film in the unexposed area. In the exposed area, photogenerated acid and heat causes hydrolysis of the ester group, enhancing the alkaline solubility of the exposed area of the film. While some lithographic properties, such as shape of the profile, linked to alkaline solubility improve as the amount of *tertiary-*butyl acrylate in the copolymer increases, other properties such as plasma-etch resistance suffer as the amount of hydroxy styrene is decreased. Moreover, as the scope of optical lithography is extended to sub-0.18 µm patterning, it is important to tailor resist materials to improve sensitivity, line edge roughness, post-exposure bake (PEB) temperature sensitivity and post-exposure delay (PED) sensitivity.

The objective of this invention is to provide novel polymers suitable for sub-0.18 µm lithography. These polymers have improved photosensitivity, line-edge roughness, PEB and PED sensitivity.

### Brief Summary of the Invention:

The polymers of this invention are useful in photoresist/radiation-sensitive compositions that include a photoacid generator, a solvent, and optionally, a basic compound.

The polymers of this invention are *tertiary*-butyl acrylate polymers comprising the monomeric units: 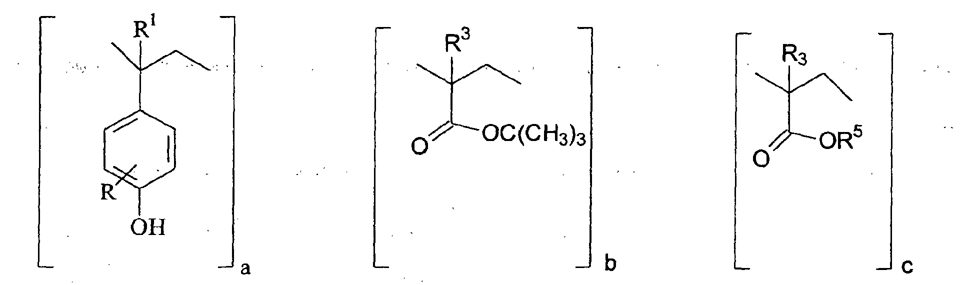 where 0.5 ≤ a ≤ 0.7, 0.15 ≤ b ≤ 0.3, 0.1 ≤ c ≤ 0.2, 0.3 ≤ b + c ≤ 0.5; R is H or a C₁-C₄ alkyl group; R¹ is H, methyl or CH₂OR²; each R³ is independently H, methyl, CH₂OR², CH₂CN, CH₂X, or CH₂COOR⁴ where X is Cl, I, Br or F; R² is H or a C₁-C₄ alkyl group; R⁴ is C₁-C₄ alkyl group; R⁵ is an isobornyl, cyclohexyl methyl, cyclohexyl ethyl, benzyl, phenethyl or tetrahydrofurfural group. Preferably, a is from 0.60 to 0.65; b is from 0.20 to 0.25; c is from 0.10 to 0.20; and b+c is from 0.35 to 0.40; R is H, R¹ is H, R² is H; each R³ is independently H or methyl; and R⁵ is an isobornyl group.

### Detailed Description of the Invention

Examples of the monomeric unit 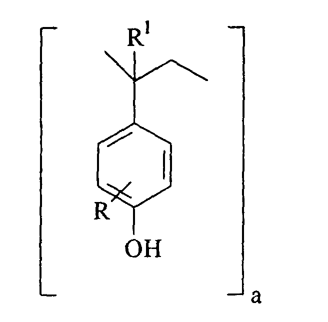 include, but are not limited to, hydroxy styrene and α-methyl hydroxy styrene.

Examples of the monomeric unit 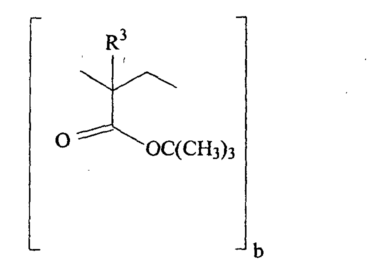 include, but are not limited to, *tertiary*-butyl acrylate, *tertiary*-butyl methacrylate, *di-tertiary*-butyl itaconate, and *tertiary*-butyl hydroxymethylacrylate.

Examples of the monomeric unit 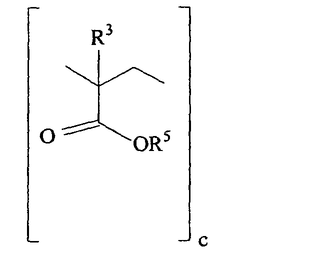 include, but are not limited to, cyclohexyl methyl (meth)acrylate, cyclohexyl ethyl (meth)acrylate, phenethyl (meth)acrylate, benzyl (meth)acrylate, isobornyl (meth)acrylate and tetrahydrofurfural (meth)acrylate.

The novel polymers of this invention can be employed in photoresist compositions, especially for compositions that are intended for use in dep UV photolithography.

The photoresist composition will include a photoacid generator (PAG). The function of the PAG is to produce an acid upon exposure to radiation/photolysis, thereby increasing alkali solubility of the polymer in a chemically amplified resist by removing acid-labile groups, thus generating an alkali soluble moiety.

Any suitable photoacid generator compound may be used in the photoresist composition. The photoacid generator compound may be, for example, onium salts such as diazonium, sulfonium, sulfoxonium and iodonium salts, and sulfone compounds, sulfonate compounds, sulfonimide compounds, diazomethane compounds, and disulfones. In addition, suitable photoacid generator compounds are disclosed in US-A-5558978, US-A- 5981140, US-A-5468589, US-A-6010098 and US-A- 6037098 which are incorporated herein by reference.

In the present invention, the photoacid generator is gererally used at about 0.5 parts to 20 parts by weight per 100 parts of polymer.

The photoresist composition of this invention also includes a solvent. The solvent should be inert, should dissolve all components in the composition, should not undergo reaction with other components, and should be removed on drying after coating. Suitable solvents include, but are not limited to organic solvents, such as 1-methoxy-2-propanol acetate (PMA), 2-methoxy-1-propylene acetate, N-methylpyrrolidone (NMP), γ butyrolactone (GBL), dimethyl-2-piperidone, diglyme, tetrahydrofuran (THF), propylene glycol monomethyl etheracetate (PGMEA), propylene glycol monomethylether (PGME), methyl ethyl ketone, methyl isobutyl ketone, 2-heptanone, cyclopentanone, cyclehexanone, 2-methoxyethanol, 2-ethoxyothanol, 2-ethoxyethyl acetate, I-methoxy-2-propyl acetate, 1,2-dimethoxy ethane ethyl acetate, cellosolve acetate, propylene glycol monoethyl ether acetate, methyl lactate, ethyl lactate, methyl pyruvate, ethyl pyruvate, methyl 3-methoxypropionate, ethyl 3-methoxypropionate, 1,4-dioxane, ethylene glycol monoisopropyl ether, diethylene glycol monoethyl ether, diethylene glycol monomethyl ether, diethylene glycol dimethyl ether, and the like.

In the present invention, the solvent is generally about 100 to 1000 parts by weight per 100 parts by weight of polymer in the photoresist composition.

Optionally, the composition of the present invention may include a basic compound. The basic compound functions to scavenge protons present in the radiation sensitive composition prior to being irradiated by the actinic radiation. The base prevents attack and cleavage of the acid labile groups by the undesirable acids thereby increasing the performance and stability of the resist. Suitable examples of basic compounds are, for example, 1,5-diazobicyclo[4.3.0]non-5-ene (DBN), 1,8-diazobicyclo[5.4.0]undec-7-ene (DBU), 2,4,5-triphenylimidazole, trimethylpropanetris(2-methyl-aziridinepropionate), 1-cyclohexyl-3-(2-morpholonoethyl)-2-thiourea, Troger's Base, 1-amino-4-piperazine, 4-(3-aminopropyl)morpholine, 2-(aminophenyl)-6-methylbenzothiazole, tribenzylamine, 1,1,4,7,10,10-hexamethyltriethylenetetramine, thiomorpholine, 1,3-bis(3-pyridylmethyl)-2-thiourea, 4,4"-tetramethylenedipiperidine, aniline, N-methylaniline, N,N-dimethylaniline, o-toluidine, m-toluidine, p-toluidine, 2,4-lutidine, quinoline, isoquinoline, formamide, N-methyl-formamide, N,N-dimethylformamide, acetamide, N-methyl-acetamide, N,N-dimethylacetamide, 2-pyrrolidone, N-methylpyrrolidone imidazole, α-picoline, β-picoline, γ-picoline, o-aminobenzoic acid, m-aminobenzoic acid, p-aminobenzoic acid, 1,2-phenylenediamine, 1,3-phenylene-diamine, 1,4-phenylenediamine, 2-quinolinecarboxylic acid, 2-amino-4-nitrophenol, and triazines such as 2-(p-chloro-phenyl)-4,6-trichloromethyl-s-triazine, 1,3,5-tribenzylhexahydro-1,3,5-triazine, tris[2-(2methoxyethoxy)ethyl]amine, t-butyl ammonium hydroxide and mixtures thereof.

In the present invention, the basic compound may be included at generally about 2 to 50 parts by weight per 100 parts by weight of the photoacid generator used in the photoresist composition.

The present invention may further include one or more other additives. Suitable additives are, for example, surfactants, adhesion promoters, leveling agents, dyes, mixtures thereof, and the like.

In addition, the present invention includes a process for forming a relief pattern using the composition of the present invention. The process comprises the steps of: (a) coating on a suitable substrate, a photoresist composition comprising a *tertiary*-butyl acrylate polymer of this invention, a photoacid generator, a solvent, and optionally a basic compound, forming a coated substrate; (b) drying the photoresist composition; (c) imagewise exposing the coated substrate to actinic radiation; (d) post exposure baking the coated substrate at an elevated temperature; (e) developing the coated substrate with an aqueous developer, forming an imaged coated substrate; and (f) rinsing the imaged coated substrate.

The invention is further described in detail by the following examples.

### Comparative Polymer Example P1

A copolymer containing 60 mole percent of hydroxy styrene and 40 mole percent of *tertiary*-butyl acrylate was purchased from TriQuest, LP.

### Comparative Polymer Example P2

### Synthesis of copolymer of hydroxy styrene and isobornyl acrylate:

A 500 mL three necked flask was fitted with a temperature probe attached to a temperature controller, an adaptor for nitrogen and a reflux condensor. The flask was placed in an oil bath over a magnetic stirrer. 20.83 G of isobornyl acrylate and 64.88 g of acetoxy styrene along with 60 g of p-dioxane and 200 g isopropyl alcohol were charged into the flask. Nitrogen gas was bubbled through the monomer solution for 30 minutes. The contents were stirred and the temperature of the reaction mixture was raised to 60°C. 2.92 G of benzoyl peroxide was charged and the reaction temperature was gradually increased to 78°C. After completion of the reaction, the reaction contents were allowed to cool to 24°C. The copolymer formed was isolated from its solution by precipitation in 2000 mL of de-ionized water. The isolated polymer was washed with 800 mL of de-ionized water, filtered and dried in a vacuum oven.

A 500 mL three-necked flask was fitted with a temperature probe attached to a temperature controller, an adaptor for nitrogen and a Dean-Stark apparatus with reflux condensor. 85 G of the dried polymer was suspended in 300 g of methanol. 5 G of 10% sodium methoxide solution in methanol was added. Temperature of the reaction mixture was raised to 66°C. Methyl acetate formed was removed by azeotropic distillation with methanol. Conversion of acetoxy styrene to hydroxy styrene was monitored by disappearance of IR peak at 1765 cm-1. After completion of the reaction, the reaction mixture was cooled to room temperature (19°C). Base-catalyst was removed by stirring the solution with 10 g of Amberlyst ion-exchange (A-15) resin for 2 hours. Ion-exchange resin was removed by filtration. Hydroxy styrene/isobornyl acrylate copolymer was isolated by precipitation in 1200 mL of de-ionized water. The isolated polymer was washed three times with 1500 mL de-ionized water, filtered and dried in a vacuum oven at 60°C for 24 hours.

The copolymer contained 79 mole% of hydroxy styrene and 21 mole% of isobornyl acrylate, as determined by 1H- and 13C-NMR. This polymer had a Mw of 14,467.

### Comparative Polymer Examples P3, P10, and P11 and Examples P4, P5, P6, P7, P8, P9, P12, and P13 of this Invention

Following the synthesis procedure described in Comparative Polymer Example P2, a series of polymers were synthesized. Different monomers were used to prepare different polymers. The polymer composition was varied by using monomers in different molar ratios in the feedstock as described in Table 1:

**TABLE 1**

| Example | Monomer composition In Feedstock | Polymer Composition ¹H & ¹³C-NMR | Mw |
|---|---|---|---|
| P3 (comparative) | Acetoxy styrene (70)/ isobornyl methacrylate (20)/t-butyl acrylate (10) | Hydroxy styrene (68)/isobornyl methacrylate (21)/t-butyl acrylate (11) | 14,800 |
| P4 | Acetoxy styrene (60)/ isobornyl acrylate (15)/t-butyl acrylate (25) | Hydroxy styrene (61)/isobornyl acrylate (13)/t-butyl acrylate (26) | 18,800 |
| P5 | Acetoxy styrene (60)/ phenethyl acrylate (15)/t-butyl acrylate (25) | Hydroxy styrene (58)/ phenethyl acrylate (23)/t-butyl acrylate (19) | 12,475 |
| P6 | Acetoxy styrene (60)/ phenethyl acrylate (5)/ isobomyl acrylate (10)/t-butyl acrylate (25) | Hydroxy styrene (62)/ phenethyl acrylate (5)/ isobornyl acrylate (16)/t-butyl acrylate (17) | 14.317 |
| P7 | Acetoxy styrene (60)/ cyclohexylethyl acrylate (5)/ isobornyl acrylate (10)/t-butyl acrylate (25) | Hydroxy styrene (60)/ phenethyl acrylate (5)/ isobornyl acrylate (10)/t-butyl acrylate (20) | 14,496 |
| P8 | Acetoxy styrene (65)/ isobornyl acrylate (12)/t-butyl acrylate (23) | Hydroxy styrene (64)/isobornyl methacrylate (13)/t-butyl acrylate (23) | 15,383 |
| P9 | Acetoxy styrene (65)/ isobornyl methacrylate (15)/t-butyl acrylate (25) | Hydroxy styrene (62)/ isobornyl methacrylate (13)/t-butyl acrylate (25) | 13,537 |
| P10 (comparative) | Acetoxy styrene (70)/ isobomyl methacrylate (10)/t-butyl acrylate (20) | Hydroxy styrene (71)/ isobornyl methacrylate (9)/t-butyl acrylate (20) | 14,407 |
| P11 (comparative) | Acetoxy styrene (70)/ isobornyl acrylate (15)/t-butyl acrylate (15) | Hydroxy styrene (71)/ isobornyl acrylate (10)/t-butyl acrylate (19) | 13,710 |
| P12 | Acetoxy styrene (65)/ isobornyl methacrylate (15)/t-butyl acrylate (20) | Hydroxy styrene (66)/ isobornyl methacrylate (15)/ t-butyl acrylate (19) | 14,322 |
| P13 | Acetoxy styrene (65)/ isobornyl acrylate (15)/t-butyl acrylate (25) | Hydroxy styrene (63)/ isobornyl acrylate (12)/t-butyl acrylate (25) | 13,977 |

### Lithographic Evaluation:

### Preparation of Photoresists Formulations:

The resist components were blended in amber colored glass bottles. All the components are the same for all the formulation, unless, stated otherwise.

All components were weighed on an electronic balance having an accuracy to □ 0.01 grams. When all the components had dissolved, the resist samples were micro filtered directly into clean bottles. Photoresist formulations are described in Table 2:

**TABLE 2**

| Formul ation type | Polymer (amount) | PAG used (amount) | Base used (amount) | Solvent (amount) |
|---|---|---|---|---|
| I | 13.2 g | Triphenylsulfonium 2,4,6-triisopropyl benzenesulfonate (0.27 g) | 2,4,5-triphenyl imidazole (0.018 g) | PGMEA (86.5 g) |
| II | 12.94 g | Triphenylsulfonium 2,4,6-triisopropyl benzenesulfonate (0.54 g) | 1,5-diazobi-cyclo [4.3.0]non-5-ene (0.019 g) | PGMEA (86.5 g) |
| III | 12.88g | Triphenylsufonium-dodecylbenzene sulfonate (0.61 g) | 1,5-diazobicyclo [4.3.0]non-5-ene (0.014 g) | PGMEA (86.5 g) |

### Coating, Baking, Exposure, Post Exposure Baking, and Developing of the Photoresists:

The following general procedure was followed for the development of a positive tone image:

The wafers were spin coated by applying 3 mL of photoresist formulations to the static six-inch wafers. The wafer was then spun to give a uniform film thickness of around 4900 Å. These photoresist coated wafers were then baked (SB) at 140°C (unless otherwise stated) for 60 seconds to remove the residual solvents. The softbaked photoresist coated wafers were then exposed using 248 nm wavelength light on an ISI XLS 0.53 NA stepper. After completion of exposure, the wafers were subjected to a post exposure bake (PEB) at 140°C (unless otherwise stated) for 60 seconds. Following the PEB, the wafers were puddle or spray-developed using a 0.262 N tetramethylammonium hydroxide, aqueous developer. A deionized water rinse was applied for 20 seconds while spinning , followed by dry nitrogen gas to dry the wafer.

### Polymer Composition and Dissolution Properties of the Resist:

Dissolution rate data were generated by static immersion development in 0.26 N TMAH using a Perkin Elmer, multiple channel Development Rate Monitor (DRM). Sixteen separate open frame exposures (zones) ranging from unexposed to 60 mJ/cm² were printed. The 256 channel raw data were reduced to 16 zones using DREAMS PC software. The results of DRM data are shown in Table 3.

**TABLE 3**

| **Resist formulation type: I (see Table 2); SB/PEB: 130/135 °C for 60 seconds** | | | |
|---|---|---|---|
| Example | Polymer Example | Polymer Composition | Dark Film Erosion (Å) |
| 1 | P1 | Hydroxy styrene (60) t-butyl acrylate (40) | 460 |
| 2 | P10 | Hydroxy styrene (71) isobornyl methacrylate(9) t-butyl acrylate (20) | 491 |
| 3 | P11 | Hydroxy styrene (7-1) isobornyl acrylate (10) t-butyl acrylate (19) | 848 |
| 4 | P9 ) | Hydroxy styrene (62) Isobornyl methacrylate(13) t-butyl acrylate (25) | 100 |
| 5 | P12 | Hydroxy styrene (66) Isobornyl methacrylate(15) t-butyl acrylate (19) | 120 |
| 6 | P13 | Hydroxy styrene (63) Isobornyl acrylate(12) t-butyl acrylate (25) | 242 |

The desirable, low dark film erosion could be obtained with terpolymers containing hydroxy styrene 60-66% and isobornyl acrylate or isobornyl methacrylate levels of 12-15%.

### Polymer Composition and Photosensitivity:

**TABLE 4**

| **Resist formulation type: I (see Table 2); SB/PEB: 130/135 °C for 60 seconds** | | | | |
|---|---|---|---|---|
| Example | Polymer Example | Polymer Composition | E0 (mJ /cm ²) | E0 pt (mJ/ cm ²) |
| 1 | P2 | Hydroxy styrene (79) Isobornyl acrylate (21) | >100 | - |
| 2 | P3 | Hydroxy styrene (68) Isobornyl methacrylate(21) t-butyl acrylate (11) | >68 | - |
| 3 | P13 | Hydroxy styrene (63) Isobornyl methacrylate(12) t-butyl acrylate (25) | 18. 5 | 34 |

The desirable photosensitivity of <40 mJ/cm² was achieved with example P13.

### Polymer Composition and Post Exposure Delay (PED) Sensitivity:

PED sensitivity was determined by measuring the 0.17-µm line-space features after 20 minutes delay between exposure and post-exposure baking (PEB) and at zero time-delay. The results are tabulated in Table 5.

**TABLE 5**

| **Formulation type: III (from table 2); SB/PEB: 140/140°C; Exposure conditions: NA: 0.53; (outer): 0.74; (inner) 0.5:** | | | | | |
|---|---|---|---|---|---|
| Example | Polymer Example | Polymer Composition | Energy to size 0.17 µ L/S (mJ/c m²) | CD (0.17 µm) at T=0 | CD (0.17 µm) At T=20 minutes After exposure |
| 1 | P2 | Hydroxy styrene (79) Isobornyl acrylate (21) | 87 | 0.15 µm | Closed |
| 2 | P3 | hydroxy styrene (68) isobornyl methacrylate(21) t-butyl acrylate (11) | 84 | 0.175 µm | Closed |
| 3 | P4 | hydroxy styrene (61) isobornyl acrylate(13) t-butyl acrylate (26) | 34 | 0.168 µm | 0.169 µm |
| 4 | P5 | hydroxy styrene (58) phenethyl acrylate (19) t-butyl acrylate (23) | 37 | 0.170 µm | 0.172 µm |

Resists based on P4 and P5 polymers do not show any change in the CD of 0.17 µm line/space pair, indicating good PED stability.

### Etch Selectivity:

Plasma etching studies were carried out on a LAM TCP-9400 etcher. The etching conditions were 700 W (source), -20°C; 200 sccm Ar, 8 sccm CF₄, 12 sccm CHF₃: pressure 700 m torr.

Substrate used: DUV-30 (Brewer Science); thickness: 60 nm on Poly-Si (100 nm)/ Resist formulation type (from Table 2): I; Resist thickness: variable; SB/PEB: 130/135 °C; Feature Type/Size: 0.25 µm isolated line. Results are shown in Table 6.

**TABLE 6**

| Example | Polymer Example | Polymer Composition | Resist Film Thickness (nm) | Resist film Thickness After etching through BARC+ Poly-Si (nm) | % Film thickness loss |
|---|---|---|---|---|---|
| 1 | P1 | hydroxy styrene (60) t-butyl acrylate (40) | 627.5 | 195 | 69 |
| 2 | P12 | hydroxy styrene (66) isobornyl methacrylate(15) t-butyl acrylate (19) | 640 | 255 | 60 |

Resist based on P10 retains 9% more film after etching through 100 nm Poly-Si and 60 nm of BARC compared to resist based on P1.

### Photoresist Evaluations:

The lithographic properties of the photoresist formulations are summarized in Table 7.

**TABLE 7**

| Example | Polymer Example | Energy to size 0.15 µm isolated line mJ/cm² | ultimate Resolution µm | Formul ation Type (from Table 2) | SB/PEB (°C) | DOF (0.15 µm isolated line) |
|---|---|---|---|---|---|---|
| 1 | P1 | 18 mJ (to size 0.18 µm) | 0.15 | I | 130/135 | 0.6 µm (0.18 µm line) |
| 2 | P4 | 30 mJ | 0.13 | II | 140/140 | 0.6 µm |
| 3 | P7 | 20 mJ | 0.13 | II | 140/140 | 0.5 µm |
| | | | | | | |

The present invention has been described with particular reference to the preferred forms thereof. It will be apparent to one of ordinary skill in the art that changes and modifications can be made thereto without departing from the spirit and scope of the present invention as defined by the following claims.

## Claims

1. A photoresist composition comprising:
a polymer of *tertiary*-butyl acrylate comprising monomeric units of: 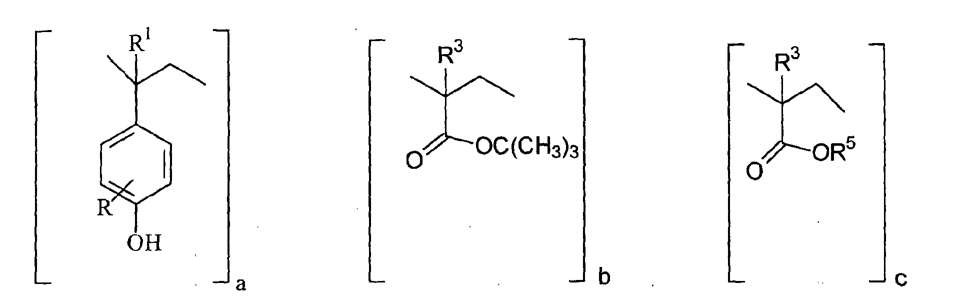 where 0.5≤a≤0.7; 0.15 ≤ b ≤ 0.3; 0.1 ≤ c ≤ 0.2; 0.3 ≤ b + c ≤ 0.5; R is selected from H, or a C₁-C₄ alkyl group; R¹ = H, methyl, CH₂OR²; each R³ is independently selected from H, methyl, CH₂OR. CH₂CN, CH₂X, or CH₂COOR⁴ group where X= Cl, I, Br,or F; R² is selected from H or C₁-C₄ alkyl; R⁴ is a C₁-C₄ alkyl group; R⁵ is selected from an isobornyl, cyclohexyl methyl, cyclohexyl ethyl, benzyl, phenethyl, or tetrahydrofurfural group; a photoacid generator; and a solvent;

2. A photoresist composition according to claim 1 also containing a basic compound.

3. A photoresist composition of Claim 1 or Claim 2 wherein R is H; R¹ is H; R² is H; each R³ is independently H or methyl; and R⁵ is isobornyl.

4. A photoresist composition of any preceding claim wherein a is from 0.60 to 0.65; b is from 0.20 to 0.25; c is from 0.10 to 0.20; and b+c is from 0.35 to 0.40.

5. A polymer of *tertiary*-butyl acrylate having the monomeric units of: 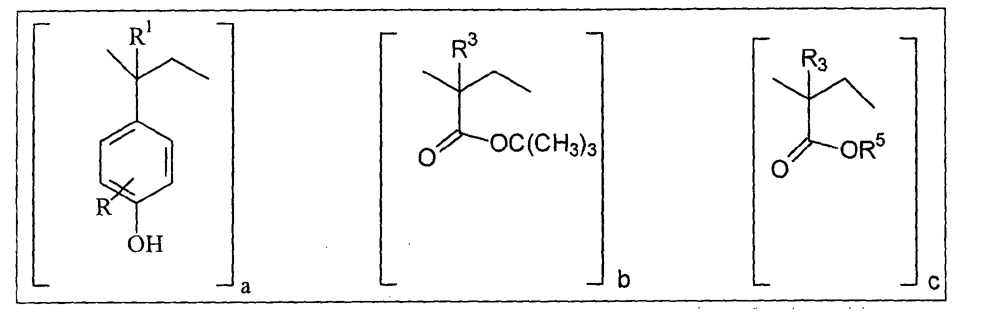 where 0.5 ≤ a ≤ 0.7; 0.15 ≤ b ≤ 0.3; 0.1 ≤ c ≤ 0.2; 0.3 ≤ b + c ≤ 0.5; R is selected from H, or a C₁-C₄ alkyl group; R¹ = H, methyl, CH₂OR²; each R³ is independently selected from H, methyl, CH₂OR. CH₂CN, CH₂X, or CH₂COOR⁴ group where X= Cl, I, Br,or F; R² is selected from H or C₁-C₄ alkyl; R⁴ is a C₁-C₄ alkyl group; R⁵ is selected from an isobornyl, cyclohexyl methyl, cyclohexyl ethyl, benzyl, phenethyl, or tetrahydrofurfural group.

6. A polymer of Claim 5 wherein R is H; R¹ is H; R² is H; each R³ is independently H or methyl; and R⁵ is isobornyl.

7. A polymer of Claim 5 or 6 wherein a is from 0.60 to 0.65; b is from 0.20 to 0.25; c is from 0.10 to 0.20; and b+c is from 0.35 to 0.40.

8. A process for forming a relief pattern, comprising the steps of:
a) coating on a substrate, the photoresist composition of any preceding claim, forming a coated substrate;
b) drying the photoresist composition;
c) imagewise exposing said coated substrate to actinic radiation;
d) post exposure baking said coated substrate at an elevated temperature;
e) developing said coated substrate with an aqueous developer, forming an imaged coated substrate; and
f) rinsing the imaged coated substrate.
